# EUROPÄISCHE PATENTANMELDUNG

(11) **EP 4 197 999 A1**
(43) Veröffentlichungstag der Anmeldung: **21.06.2023**
(21) Anmeldenummer: 21215349.8
(22) Anmeldetag: 17.12.2021
(51) Int. Cl.: C07C 45/00, C07F 15/00

(54) **REAKTIONSGEMISCH MIT PT-BIPHENYL-CHLOR-KOMPLEX**

(71) Anmelder: Evonik Operations GmbH, 45128 Essen (DE)
(72) Erfinder: SCHNEIDER, Carolin, 40789 Monheim am Rhein (DE); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(57) **Zusammenfassung**

Reaktionsgemisch mit einem Pt-Biphenyl-Chlor-Komplex, sowie dessen Verwendung zur Katalyse einer Hydroformylierungsreaktion.

## Beschreibung

Die vorliegende Erfindung betrifft ein Reaktionsgemisch mit einem Pt-Biphenyl-Chlor-Komplex, sowie dessen Verwendung zur Katalyse einer Hydroformylierungsreaktion.

In EP 2663573 B1 wird ein Verfahren zur Herstellung von (**1**) beschrieben.

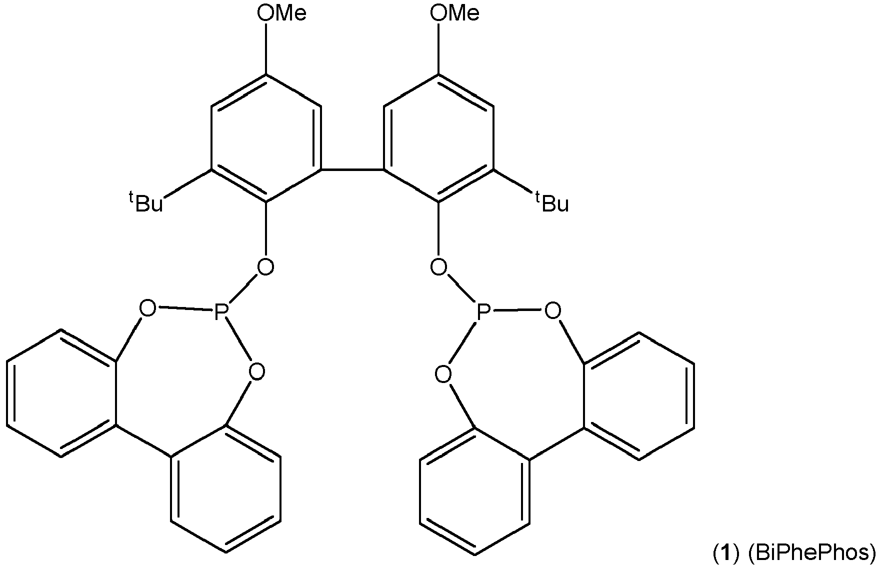

Der vorliegende Erfindung lag die Aufgabe zugrunde, ein neues Reaktionsgemisch bereitzustellen. Das Reaktionsgemisch soll hierbei bei der Katalyse von Hydroformylierungsreaktionen eine gesteigerte Ausbeute liefern.

Diese Aufgabe wird gelöst durch eine Reaktionsgemisch gemäß Anspruch 1.

Reaktionsgemisch umfassend ein Lösungsmittel und einen Komplex umfassen:
a) Pt;
b) einen Liganden entsprechend der Formel (**I**): 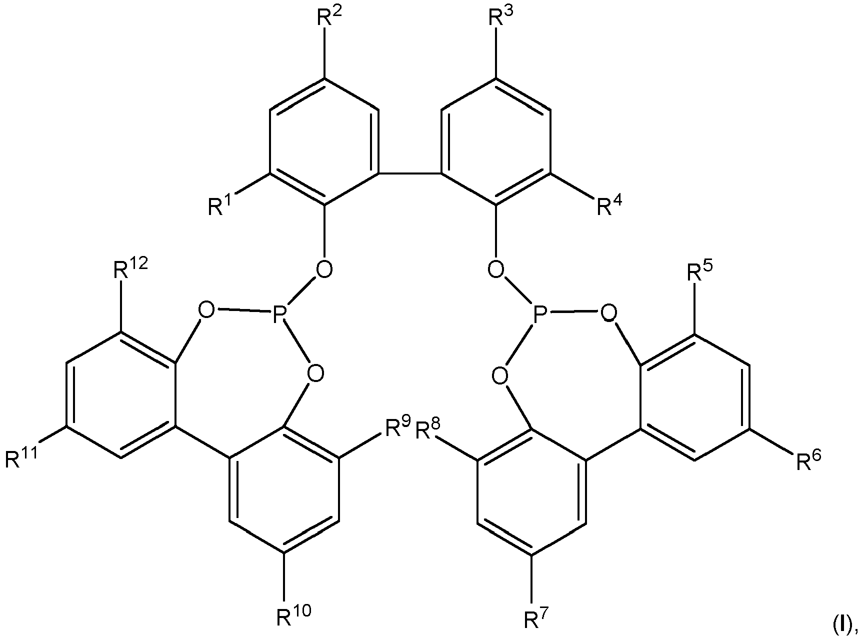 wobei R¹, R², R³, R⁴, R⁵, R⁶, R⁷, R⁸, R⁹, R¹⁰, R¹¹, R¹² ausgewählt sind aus: -H, -(C₁-C₁₂)-Alkyl, -O-(C₁-C₁₂)-Alkyl, -(C₆-C₂₀)-Aryl;
c) einen Chlor-Liganden,
wobei das Reaktionsgemisch frei von Sn ist.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, *n*-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Die Definition von (C₁-C₁₂)-Alkyl gilt analog auch für das (C₁-C₁₂)-Alkyl in -O-(C₁-C₁₂)-Alkyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

In einer Ausführungsform sind R¹, R², R³, R⁴ ausgewählt aus: -(C₁-C₁₂)-Alkyl, -O-(C₁-C₁₂)-Alkyl.

In einer Ausführungsform stehen R¹ und R⁴ für -(C₁-C₁₂)-Alkyl.

In einer Ausführungsform stehen R¹ und R⁴ für -^{t}Bu.

In einer Ausführungsform stehen R² und R³ für -O-(C₁-C₁₂)-Alkyl.

In einer Ausführungsform stehen R² und R³ für-OMe.

In einer Ausführungsform stehen R⁵, R⁶, R⁷, R⁸, R⁹, R¹⁰, R¹¹, R¹² für -H.

In einer Ausführungsform stehen weist die Verbindung gemäß der Formel (**I**) die Struktur (**1**) auf:

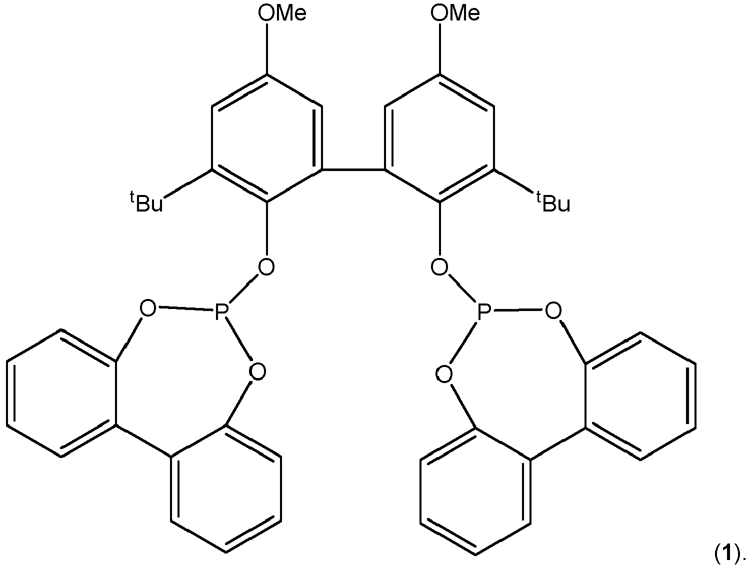

In einer Ausführungsform weist der Komplex genau einen Liganden entsprechend der Formel (**I**) auf.

In einer Ausführungsform weist der Komplex mindestens zwei Chlor-Liganden auf.

In einer Ausführungsform weist der Komplex genau zwei Chlor-Liganden auf.

In einer Ausführungsform ist das Lösungsmittel ausgewählt aus: THF, DCM, ACN, Heptan, DMF, Toluol, Texanol, Pentan, Hexan, Octan, Isooctan, Decan, Dodecan, Cyclohexan, Benzen, Xylene, Marlotherm, Propylencarbonat, MTBE, Diglyme, Triglyme, Diethylether, Dioxan, Isopropanol, Tertbutanol, Isononanol, Isobutanol, Isopentanol, Ethylacetat.

In einer Ausführungsform ist das Lösungsmittel ausgewählt aus: THF, DCM, ACN, Heptan, DMF, Toluol, Texanol.

Neben dem Reaktionsgemisch an sich, wird auch dessen Verwendung zur Katalyse einer Hydroformylierungsreaktion beansprucht.

Verwendung eines zuvor beschriebenen Reaktionsgemisches zur Katalyse einer Hydroformylierungsreaktion.

Im Folgenden soll die Erfindung anhand von Ausführungsbeispielen näher erläutert werden.

### Versuchsbeschreibung

Ein Vial wurde mit PtCl₂, Ligand, und einem im Ofen getrockneten Rührstab bestückt. Dann wird das Vial mit Septum (PTFE-beschichteter StyrolButadien-Kautschuk) und Phenolharzdeckel verschlossen. Das Vial wird dreimal evakuiert und mit Argon wieder befüllt. Mit einer Spritze wurden Toluol und Methyl-9-decenoat in das Vial gegeben. Das Vial wurde in eine Legierungsplatte gestellt, die in einen Autoklav der Reihe 4560 von Parr Instruments unter Argon Atmosphäre überführt wurde. Nach dreimaligem Spülen des Autoklaven mit CO/H₂ wurde der Synthesegasdruck auf 40 bar bei Raumtemperatur erhöht. Die Reaktion wurde 20 h bei 120 °C durchgeführt. Nach Beendigung der Reaktion wurde der Autoklav auf Raumtemperatur abgekühlt und vorsichtig entspannt. Der Ausbeute und Selektivität wurden durch GC-Analyse bestimmt.

### Hydroformylierung von Methyl-9-decenoat (9-Dame)

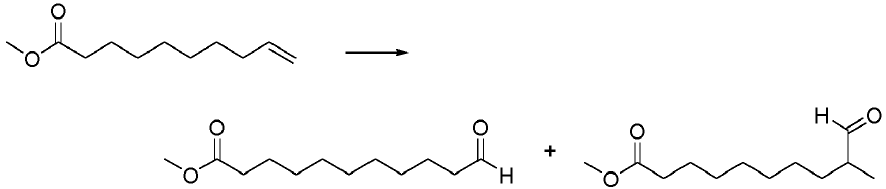

### Reaktionsbedingungen:

20.0 mmol Methyl-9-decenoat (9-Dame), 0.2 mol% PtCl₂, 5.0 Äquivalente Ligand (1), Lösungsmittel: Toluol, p(CO/H₂): 40 bar, T: 120 °C, t: 20 h.

### Reaktionsbedingungen Vergleichsversuch:

20.0 mmol Methyl-9-decenoat (9-Dame), 0.2 mol% PtCl₂, 0.2 mol% SnCl₂, 5.0 Äquivalente Ligand (1), Lösungsmittel: Toluol, p(CO/H₂): 40 bar, T: 120 °C, t: 20 h.

Ausbeuten:
PtCl₂: 77%
PtCl₂ + SnCI2: 12 %

Wie die Versuchsergebnisse zeigen, wird die Aufgabe durch den erfindungsgemäßen Komplex gelöst.

## Patentansprüche

1. Reaktionsgemisch umfassend ein Lösungsmittel und einen Komplex umfassen:
a) Pt;
b) einen Liganden entsprechend der Formel (**I**): 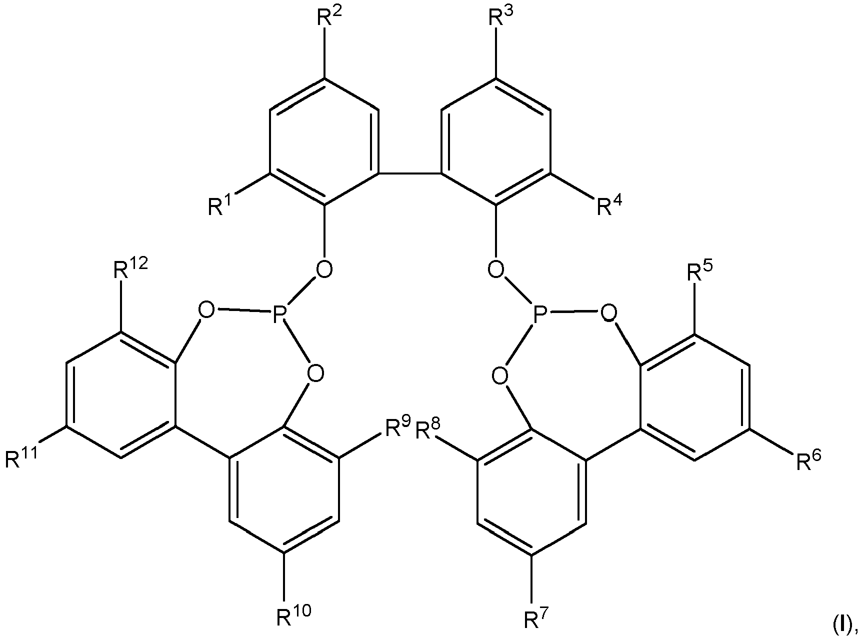 wobei R¹, R², R³, R⁴, R⁵, R⁶, R⁷, R⁸, R⁹, R¹⁰, R¹¹, R¹² ausgewählt sind aus: -H, -(C₁-C₁₂)-Alkyl, -O-(C₁-C₁₂)-Alkyl, -(C₆-C₂₀)-Aryl;
c) einen Chlor-Liganden,
wobei das Reaktionsgemisch frei von Sn ist.

2. Reaktionsgemisch nach Anspruch 1,
wobei R¹, R², R³, R⁴ ausgewählt sind aus: -(C₁-C₁₂)-Alkyl, -O-(C₁-C₁₂)-Alkyl.

3. Reaktionsgemisch nach einem der Ansprüche 1 oder 2,
wobei R¹ und R⁴ für -(C₁-C₁₂)-Alkyl stehen.

4. Reaktionsgemisch nach einem der Ansprüche 1 bis 3,
wobei R² und R³ für -O-(C₁-C₁₂)-Alkyl stehen.

5. Reaktionsgemisch nach einem der Ansprüche 1 bis 4,
wobei R⁵, R⁶, R⁷, R⁸, R⁹, R¹⁰, R¹¹, R¹² für -H stehen.

6. Reaktionsgemisch nach einem der Ansprüche 1 bis 5,
wobei die Verbindung gemäß der Formel (**I**) die Struktur (**1**) aufweist:

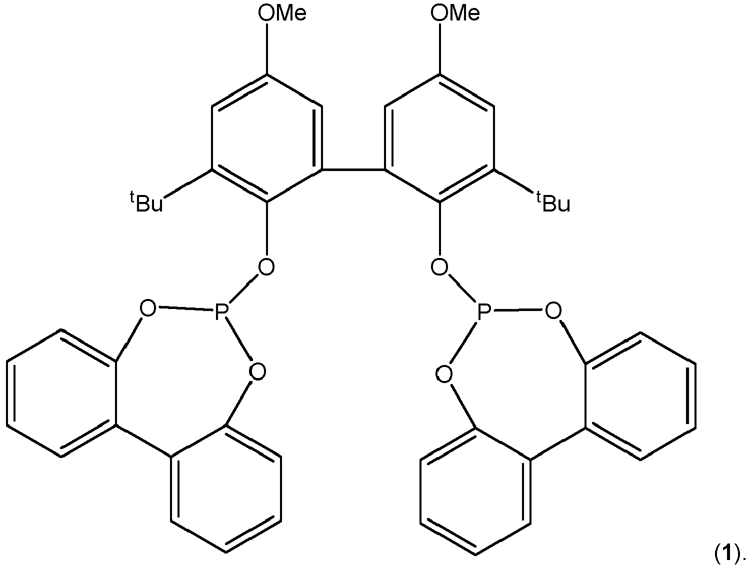

7. Reaktionsgemisch nach einem der Ansprüche 1 bis 6,
wobei der Komplex genau einen Liganden entsprechend der Formel (**I**) aufweist.

8. Reaktionsgemisch nach einem der Ansprüche 1 bis 7,
wobei der Komplex mindestens zwei Chlor-Liganden aufweist.

9. Reaktionsgemisch nach Anspruch 8,
wobei der Komplex genau zwei Chlor-Liganden aufweist.

10. Reaktionsgemisch nach einem der Ansprüche 1 bis 9,
wobei das Lösungsmittel ausgewählt ist aus: THF, DCM, ACN, Heptan, DMF, Toluol, Texanol, Pentan, Hexan, Octan, Isooctan, Decan, Dodecan, Cyclohexan, Benzen, Xylene, Marlotherm, Propylencarbonat, MTBE, Diglyme, Triglyme, Diethylether, Dioxan, Isopropanol, Tertbutanol, Isononanol, Isobutanol, Isopentanol, Ethylacetat.

11. Reaktionsgemisch nach einem der Ansprüche 1 bis 10 zur Katalyse einer Hydroformylierungsreaktion.

## Geänderte Patentansprüche

### Geänderte Patentansprüche gemäss Regel 137(2) EPÜ.

1. Reaktionsgemisch zur Katalyse einer Hydroformylierungsreaktion umfassend ein Lösungsmittel und einen Komplex umfassen:
a) Pt;
b) einen Liganden entsprechend der Formel (I): 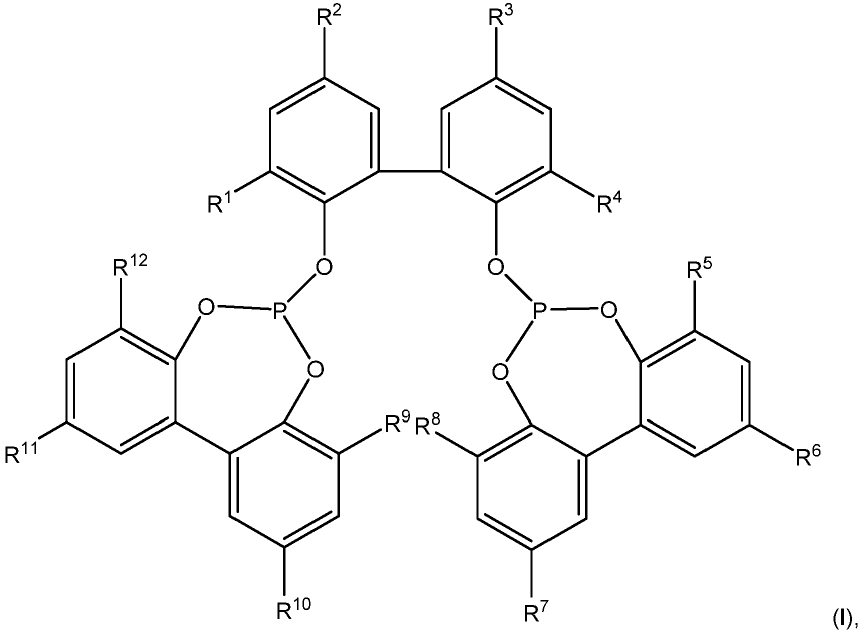 wobei R¹, R², R³, R⁴ ausgewählt sind aus: -(C₁-C₁₂)-Alkyl, -O-(C₁-C₁₂)-Alkyl und R⁵, R⁶, R⁷, R⁸, R⁹, R¹⁰, R¹¹, R¹² ausgewählt sind aus: -H, -(C₁-C₁₂)-Alkyl, -Q-(C₁-C₁₂)-Alkyl, - (C₆-C₂₀)-Aryl;
c) einen Chlor-Liganden,
wobei das Reaktionsgemisch frei von Sn ist.

2. Reaktionsgemisch nach Anspruch 1,
wobei R¹ und R⁴ für -(C₁-C₁₂)-Alkyl stehen.

3. Reaktionsgemisch nach einem der Ansprüche 1 oder 2,
wobei R² und R³ für -O-(C₁-C₁₂)-Alkyl stehen.

4. Reaktionsgemisch nach einem der Ansprüche 1 bis 3,
wobei R⁵, R⁶, R⁷, R⁸, R⁹, R¹⁰, R¹¹, R¹² für -H stehen.

5. Reaktionsgemisch nach einem der Ansprüche 1 bis 4,
wobei die Verbindung gemäß der Formel (I) die Struktur (1) aufweist:

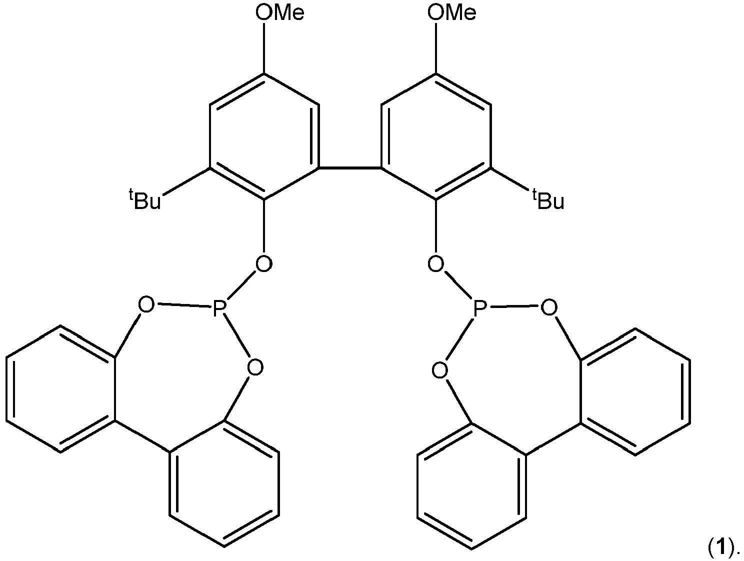

6. Reaktionsgemisch nach einem der Ansprüche 1 bis 5,
wobei der Komplex genau einen Liganden entsprechend der Formel (I) aufweist.

7. Reaktionsgemisch nach einem der Ansprüche 1 bis 6,
wobei der Komplex mindestens zwei Chlor-Liganden aufweist.

8. Reaktionsgemisch nach Anspruch 7,
wobei der Komplex genau zwei Chlor-Liganden aufweist.

9. Reaktionsgemisch nach einem der Ansprüche 1 bis 8,
wobei das Lösungsmittel ausgewählt ist aus: THF, DCM, ACN, Heptan, DMF, Toluol, Texanol, Pentan, Hexan, Octan, Isooctan, Decan, Dodecan, Cyclohexan, Benzen, Xylene, Marlotherm, Propylencarbonat, MTBE, Diglyme, Triglyme, Diethylether, Dioxan, Isopropanol, Tertbutanol, Isononanol, Isobutanol, Isopentanol, Ethylacetat.
